# FASCICULE DE BREVET EUROPEEN

(11) **EP 1 032 372 B1**
(45) Date de publication et mention de la délivrance du brevet: **02.10.2002**
(21) Numéro de dépôt: 98954686.6
(22) Date de dépôt: 02.12.1998
(51) Int. Cl.: A61K 9/20

(54) **MASQUANT POUR GOUTS PHARMACEUTIQUES EN POUDRE**
GESCHMACKABDECKENDE PULVER FÜR PHARMAZEUTIKA
MASKING AGENT IN POWDER FORM FOR PHARMACEUTICAL FLAVOURS

(30) Priorité: 03.12.1997 EP 97810941
(43) Date de publication de la demande: 06.09.2000
(73) Titulaire: Pancosma S.A. pour l'Industrie des Produits Biochimiques, 1218 Le Grand-Saconnex (CH)
(72) Inventeur: FELISAZ, Denis, F-74100 Annemasse (FR); JACQUIER, Yvan, CH-1206 Genève (CH)
(74) Mandataire: Moinas, Michel
(86) Numéro de dépôt international: IB9801915
(87) Numéro de publication internationale: WO99027915

(56) Documents cités:
- DE-A- 19 617 487
- DE-A- 19 639 343

## Description

La présente invention se rapporte à un masquant en poudre destiné à masquer les goûts pharmaceutiques désagréables des médicaments quand cela s'avère nécessaire .

Traditionnellement, on utilise des masquants pour goûts pharmaceutiques désagréables lorsque cela s'avère indispensable pour que le patient, surtout s'il s'agit d'un enfant, accepte de prendre le médicament par voie orale. Les substances utilisées sont de natures diverses et généralement ajoutées à la formulation pharmaceutique sous forme d'excipient. On citera par exemple les édulcorants, dont la saccharine et ses dérivés.

Les nombreuses études menées sur le sujet visent plus à découvrir et à mettre en évidence de nouvelles substances qu'à la façon dont elles peuvent entrer dans la composition pharmaceutique finale. On citera par exemple de nombreux brevets, EP 0 351 566, EP 0 350 667, EP 0 351 567, portant sur un modificateur de goût tiré d'un végétal *Curculigo latifolia* appelé curculine, ou EP 0 753 296 qui indique comment rendre organoleptiquement acceptable un anti-inflammatoire stéroïdien, l'ibufène, dont le goût amer est particulièrement prononcé.

Or, le principal problème n'est pas tant de trouver de nouvelles substances chimiques , naturelles ou non, masquant les goûts désagréables que de mettre au point la forme où ces masquants, nouveaux ou non, seront utilisés.

Il importe en effet que l'effet masquant soit constant et uniforme d'une prise de médicament à l'autre, faute de quoi l'effet recherché auprès du patient ne serait pas garanti. Or tel n'est précisément pas le cas avec les masquants traditionnels qui sont soit des poudres réparties statistiquement dans la formulation médicamenteuse finale, soit des mélanges statistiques de poudres.

C'est le cas en particulier quand le masquant comprend un édulcorant comme agent de sapidité et un potentiateur de granulométries très différentes, à savoir une poudre d'un édulcorant d'une granulométrie relativement grossière et mélangée à un potentiateur se présentant sous forme d'une poudre beaucoup plus fine. L'édulcorant, c'est-à-dire l'agent qui apporte le goût sucré est habituellement de la saccharine ou du saccharinate de sodium ou de calcium, qui ont par elles-mêmes un goût amer ou métallique, parfois d'autres édulcorants intenses d'origine naturelle ou artificielle.

Le rôle premier de l'édulcorant ou plus généralement de l'agent de sapidité, est de supplanter le goût pharmaceutique désagréable du médicament final.

Le rôle du potentiateur est double. D'abord, comme son nom l'indique, il a comme effet de prolonger la perception du goût de l'agent de sapidité qui, en son absence, serait trop fugitive. Un bon potentiateur, tels que ceux utilisés classiquement, permet par exemple d'augmenter la perception, notamment la perception sucrée, dans le temps (4 à 5 fois). L'autre effet du potentiateur, et non des moindres, est de masquer les goûts secondaires ou parasites de Fédulcorant, par exempte de masquer le goût amer et métallique de la saccharine ou dé ses sels de sodium et de calcium.

Afin de lever toute ambiguïté dans la terminologie utllisée ci-après, nous utiliserons le terme de "masquant" pour désigner le mélange agent de sapidité/potentiateur, objet de l'invention, ainsi que les produits traditionnels correspondants qui serviront de comparaison, tandis que nous utiliserons l'expression "formulation masquante" ou "prémélange masquant", lorsque le masquant proprement dit est dispersé dans un support.

Les masquants traditionnels du type agent de sapidité/potentiateur sont des mélangea statistiques d'un ou plusieurs édulcorants et d'un ou plusieurs potentiateurs dont comme déjà indiqué plus haut, les granulométries sont très différentes. Malgré le soin que l'on peut apporter à leur réalisation, il ne peut en résulter que des mélangea présentant une forte hétérogénéité. Pour cette raison, nous les qualifierons par la suite de "masquants grossiers".

DE 196 17 481 décrit une composition de granulométrie homogène qui est ensuite transformée en tablettes.

Les problèmes évoqués ci-dessus sont résolus par l'invention, de façon étonnamment simple, l'invention consistant à proposer un masquant en poudre pour goûts pharmaceutiques, qui comprend un mélange entre un agent de sapidité et un potentiateur. Ce mélange se présente sous forme d'un mélange intime dont la répartition agent de sapidité/potentiateur est sensiblement homogène, non statistique, et dont la proportion de l'agent de sapidité par rapport au potentiateur est sensiblement constante et égale d'une particule de poudre à l'autre.

Le masquant en poudre selon l'invention a une granulométrie comprise entre 10 et 100 µm, avec une répartition gaussienne.

De préférence, la proportion agent de sapidité/potentiateur(s) est comprise entre 97/3 et 90/10, valeurs exprimées en parties pondérales.

Quand on parie "d'un agent de sapidité", ou "d'un potentiateur, il faut comprendre qu'on entend désigner "au moins un agent de sapidité", respectivement "au moins un potentiateur".

Comme indiqué plus haut et comme on le verra plus loin, puisque la proportion de l'agent de sapidité par rapport à l'édulcorant est sensiblement constante et égale d'une particule à l'autre, elle se révélera constante d'un granulé à l'autre dans la formulation masquante dans laquelle le masquant aura été dispersé sur un support, et en conséquence dans le médicament final. On garantira ainsi au patient, non seulement un goût acceptable, mais surtout constant d'une prise de médicament à l'autre.

Selon une des formes d'exécution préférée de l'invention, le masquant selon l'invention peut se présenter sous le forme d'une poudre ayant une répartition gaussienne et un diamètre moyen de particules compris entre 10 et 100 µm, comprenant entre 65'000'000 et 85'000'000, par exemple autour de 75'000'000, de particules par gramme, à comparer avec une saccharine de 40-80 Mesh ayant une granulométrie comprise entre 100 et 1000 µm et ne comprenant que de 50'000 à 70'000, en moyenne 60'000 particules environ au gramme.

Comme agent de sapidité, on peut utiliser un édulcorant ou d'autres substances telles que des méthyl-, éthyl- ou carboxyl(m)éthylcelluloses, des pectines, des carraghénates ou des alginates, toutes susceptibles de jouer un rôle au niveau des papilles gustatives. On utilisera avantageusement un ou plusieurs édulcorants, par exemple le saccharinate de sodium ou de calcium et la saccharine déjà cités, ainsi que d'autres édulcorants tels que l'aspartyl-phénylalanine, l'acésulfame et les cyclamates et le stévioside qui est un glycoside d'origine naturelle.

Comme potentiateur on peut utiliser la thaumatine, qui est une protéine d'origine végétale, des gtycosides tels que la néohespéridine dihydrochalcone (NHDC). la glycyrrhizine, te glutamate, etc., seuls ou en mélanges. Ces substances assurent une très grande rémanence au goût sucré et couvrent bien l'arrière-goût amer et métallique de la saccharine et de ses sels.

Le masquant en poudre selon l'invention peut contenir également d'autres ingrédients en petites quantités, tels que des arômes, des exhausteurs de goût, glutamate de sodium, nucléotides (inosinate et guanylate de sodium), maltol et éthylmaftol, etc. Ces ingrédients font partie des "micro-ingrédients" qui peuvent entrer dans la formulation intermédiaire du médicament ou prémix qui peut contenir aussi des vitamines, des oligo-éléments, voire d'autres substances médicamenteuses dispensés à titre préventif ou prophylactique. Ces dernières substances sont généralement calculées pour être présentes à raison de 75 à 150 g par kg dans le médicament final.

Le masquant selon l'invention peut être utilisé avec un grand nombre de produits pharmaceutiques ou thérapeutiques ayant des goûts déplaisants, appartenant à toutes sortes de catégories telles que par exemple les antibiotiques, analgésiques, anti-tussifs, anti-paludéens, anti-inflammatoires, anti-nauséeux, antiasthmatiques, laxatifs, expectorants, décongestants, hypo-chlolestérolémiants, antihistaminiques, etc., ou encore à des produits alimentaires à caractère diététique, pharmaceutique ou parapharmaceutique, tels que des suppresseurs d'appétit, des compléments nutritionnels ou vitaminés, etc. Pour de plus amples détails, on pourra se référer à EP 0 371 584 cité plus haut qui donne une liste importante, bien que non exhaustive, de produits pharmaceutiques dont les goûts désagréables sont à masquer.

Le masquant en poudre selon l'invention peut être préparé très simplement, selon une technologie bien connue en alimentation humaine et dans la technologie de fabrication des arômes, par "micronisation", c'est-à-dire par atomisation et séchage d'un mélange liquide, pulvérisé dans un appareil approprié. Cet appareil peut prendre la forme d'une tour dans laquelle le mélange liquide est admis au sommet par une buse ou une turbine, la poudre atomisée obtenue étant récoltée à la base de la tour. Dans certains cas, si cela s'avère nécessaire, la tour peut être remplie d'un gaz inerte pour éviter les phénomènes d'oxydation.

Le masquant en poudre selon l'invention est utilisé en prenant soin d'en faire un mélange homogène avec le principe actif et l'excipient qui généralement l'accompagne. Sur la base des valeurs usuelles, cela va permettre de constituer un médicament final dont la quantité d'édulcorant est de l'ordre de 30 à 90 g/kg et la quantité de potentiateur de 1,5 à 6,3 g/kg sur la base d'une dose moyenne de principe actif de 20 à 70 g/kg, valeurs évidemment extrêmement variables selon la composition du masquant et le principe actif considéré. Il faut bien comprendre que, puisque le masquant selon l'invention est parfaitement homogène et que la granulométrie de ce masquant est suffisamment fine, il n'y aura évidemment aucune difficulté à obtenir une formulation masquante, ou un médicament final, eux aussi parfaitement homogènes.

Selon une autre forme d'exécution, l'invention se rapporte à un médicament pulvérulent qui se présente sous forme d'un mélange intime dont la répartition agent de sapidité/potentiateur/principe actif est sensiblement homogène, non statistique, et dont les proportions agent de sapidité/potentiateur/principe actif sont sensiblement constantes et égales d'une particule de poudre à l'autre.

Un tel médicament est préparé en mélangeant en solution un agent de sapidité, un potentiateur et un principe actif, puis en pulvérisant ce mélange par atomisation séchage pour obtenir une poudre.

A partir de ce médicament "brut", on peut alors préparer, si désiré et selon des techniques classiques, un médicament final se présentant sous la forme de granulés, poudres effervescentes, cachets, pastilles et gommes à mâcher, voire sirops.

L'invention sera mieux comprise en référence aux exemples ci-après, donnés à titre non limitatif. Ces exemples seront suivis d'exemples comparatifs destinés à mieux mettre en évidence les avantages de l'invention qui sont :
- un pouvoir masquant régulier,
- la possibilité d'additionner des adjuvants en quantité elle aussi régulière
- une excellente stabilité à la conservation, meilleure que celle des masquants grossiers dont les deux poudres constitutives, de granulométries très différentes, ont au fil du temps tendance à la ségrégation ou à la sédimentation.
- le fait que, par sa structure cristalline amorphe et son taux d'humidité de 2 à 4 %,(contre 5 à 15 % pour le saccharinate de sodium ), le masquant selon l'invention est libre d'écoulement ; il n'est donc pas nécessaire d'ajouter d'antimottants tels que la silice et de ce fait, il est totalement soluble dans l'eau sans laisser de résidus, et sa solubilité est instantanée.

### EXEMPLES 1 A 9

On prépare une solution d'un ou plusieurs édulcorants en introduisant 900 litres d'eau dans une cuve, puis en chauffant jusqu'à 60°C. On ajoute ensuite lentement 1000 kg de ou des édulcorants, on agite la solution pendant 30 minutes jusqu'à la dissolution complète (la solution devient transparente ).

Parallèlement, dans une petite cuve remplie de 100 litres d'eau chauffée jusqu'à 70°C, on ajoute lentement le ou les potentiateurs, en quantité correspondant à la proportion édulcorant/potentiateur désirée pour le masquant en préparation, puis on laisse dissoudre le produit pendant 30 minutes.

On verse la solution de potentiateur(s) lentement dans la solution d'édulcorant(s)

Lorsque la solution devient transparente, on procède à l'opération d'atomisation-séchage (spray drying) dans une tour dont les conditions de travail sont les suivantes :

| | |
|---|---|
| Température de la solution | 60°C |
| Température d'air entrée | 190°C |
| Température d'air sortie | 100°C |
| Vitesse de rotation de la turbine d'atomisation | 15'000 t/min. à 20'000 t/min, |

la turbine pouvant être remplacée par une buse d'atomisation.

On obtient ainsi un masquant selon l'invention, se présentant sous forme d'une poudre fine homogène ayant une granulométrie à répartition gaussienne comprise entre 10 et 100 µm avec une moyenne de 48,5 µm et comprenant autour de 75'000'000 particules ou grains par gramme. Cette poudre est soluble dans l'eau.

On a ainsi réalisé les masquants en poudre suivants, selon les édulcorants et les potentiateurs utilisés, et selon leur proportion exprimée en valeurs pondérales :

| **EXEMPLES** | **EDULCORANTS** | **POTENTIATEURS** | **PROPORTIONS** |
|---|---|---|---|
| 1 | Saccharinate de sodium / Aspartame | NHDC | 70/25/5 |
| 2 | Saccharinate de sodium / Acésulfame K | NHDC | 35/60/5 |
| 3 | Aspartame/ Acésulfame K | NHDC | 10/86/4 |
| 4 | Saccharinate de sodium / Aspartame | NHDC / Glycyrrhizine | 70/25/2/3 |
| 5 | Saccharinate de sodium / Acésulfame K | Glycyrrhizine / Maltol | 40/53/3/4 |
| 6 | Saccharinate de sodium / Acésulfame K | Glycyrrhizine / Ethyl-Maltol | 42/53/3/2 |
| 7 | Saccharinate de sodium / Acésulfame K | NHDC / Glycyrrhizine / Ethyl-Maltol | 40/53/2/3/2 |
| 8 | Acésulfame K | NHDC / Glycyrrhizine | 95/2/3 |
| 9 | Aspartame/ Acésulfame K | NHDC / Glycyrrhizine / Ethyl-Maltol | 10/84/2/2/2 |

### EXEMPLES 10 A 12 ET EXEMPLE COMPARATIF

On prépare par mélange à sec des comprimés et poudres effervescentes de paracétamol, un antalgique antipyrétique qui a un goût amer. Chaque comprimé contient 500 mg de paracétamol en comprimés de 0,6 g ou 150 mg en poudre effervescente de 0,3 g par sachet, à l'aide des masquants ci-dessus des exemples 1, 5 et 9, et respectivement à l'aide de saccharinate de sodium 40-80 Mesh

| | **Ex. 10** % | **Ex. 11** % | **Ex. 12** % | **Ex. 13** % |
|---|---|---|---|---|
| Paracétamol | 96,15 | 94,20 | 92,30 | 96,15 |
| Exemple 1 | 3,85 | | | |
| Exemple 5 | | 5,80 | | |
| Exemple 9 | | | 7,70 | |
| Exemple comparatif: Saccharinate de sodium 40-80 Mesh | | | | 3,85 |

On procède alors à un test gustatif de chacun de ces mélanges dans 30 ml d'eau.
Exemple 10 - Contient 500 mg de paracétamol + 20 mg du masquant
Exemple 11 - Contient 500 mg de paracétamol + 30 mg du masquant
Exemple 12 - Contient 500 mg de paracétamol + 42 mg du masquant
Exemple comparatif - Contient 500 mg de paracétamol + 20 mg de saccharinate de sodium 40-80 Mesh

Les résultats obtenus sont les suivants :

Exemple 10 : goût sucré, suivi d'une légère amertume qui apparaît après 30 secondes et disparaît après 2 minutes ; puis le goût sucré continue et disparaît après 3 minutes 30 secondes.

Dans cet exemple, on trouve environ 75'000'000 de particules par g. Dans un comprimé de 0.6 g il y a donc 1'500'000 particules, d'où un effet enrobant très important vis à vis des particules du principe actif

Exemple 11: goût sucré assez proche de celui du saccharose suivi d'une légère amertume qui apparaît après 45 secondes et disparaît après 1 minute 45 secondes ; puis le goût sucré continue et disparaît après 3 minutes 6 secondes.

Dans cet exemple, on trouve aussi environ 75'000'000 particules par g. Dans un comprimé de 0,6 g, il y a donc 2'250'000 particules.

Exemple 12 : goût sucré très proche de celui du saccharose suivi d'une très légère amertume qui apparaît après 80 secondes et disparaît après 1 minute 40 secondes ; puis le goût sucré continue et disparaît après 4 minutes 25 secondes

Dans cet exemple, on trouve encore 75'.000'000 particules par g. Dans un comprimé de 0,6 g, il y a 31'500'.000 particules.

Exemple comparatif: goût sucré et amer ; l'amertume se manifeste après 10 secondes, puis le goût sucré disparaît après 1 minute et l'amertume persiste pendant 2 minutes 15 secondes.

Dans cet exemple, avec un masquant traditionnel, on trouve environ 60'000 particules par g, soit 1'200 particules par comprimé de 0,.6 g, d'où le faible effet masquant vis à vis du principe actif.

Cette comparaison démontre clairement les effets des masquants selon l'invention, comparativement à un édulcorant traditionnel à base de saccharinate de sodium.

### EXEMPLE 13

On procède comme décrit aux exemples précédents, mais en substituant du chlorhydrate de quinine et en utilisant le masquant de l'exemple 6 au paracétamol pour obtenir un comprimé contenant 500 mg de chlorhydrate de quinine et 36,48 mg de masquant.

A la dégustation, on note un goût sucré accompagné d'une flaveur de réglisse et de caramel et d'une petite amertume. ; cette amertume diminue et disparaît après 3 minutes 30 secondes et le goût sucré disparaît après 3 minutes 45 secondes.

### EXEMPLES 14 A 20

Les mélanges des exemples 10 à 12 (et de l'exemple comparatif) sont des mélanges statistiques. Pour obtenir des mélanges non statistiques et par conséquent optimalise l'effet masquant, tous les constituants qui constituent ces mélanges (édulcorant, potentiateurs, principes actifs). sont, au départ, liés intimement en une micro particule selon la même technologie de fabrication que pour les masquants selon l'invention.

On prépare ainsi une solution d'un ou plusieurs édulcorants en introduisant 900 litres d'eau dans une cuve, puis en chauffant jusqu'à 60°C On ajoute ensuite lentement 1000 kg de ou des édulcorants, on agite la solution pendant 30 minutes jusqu'à la dissolution complète (la solution devient transparente).

Parallèlement, dans une petite cuve remplie de 100 litre d'eau chauffée jusqu'à 70°C, on ajoute lentement le ou les potentiateurs, en quantité correspondant à la proportion édulcorant/potentiateur désirée pour le masquant en préparation, puis on laisse dissoudre le produit pendant 30 minutes.

On ajoute enfin le principe actif, préalablement solubilisé dans une quantité suffisante d'eau, puis on atomise le mélange dans une tour d'atomisation, dans les conditions des exemples précédents.

On procède ainsi avec du chlorhydrate de quinine, un antimalaria qui a un goût amer très prononcé.

| | **Exemple 14 %** | **Exemple 15 %** | **Exemple 16 %** |
|---|---|---|---|
| Chlorhydrate de Quinine | 95,5 | 92,8 | 93,2 |
| Masquant de l'exemple 1 | 4,6 | | |
| Masquant de l'exemple 3 | | 7,2 | |
| Masquant de l'exemple 6 | | | 6,8 |
| Les % de masquant de l'ex. 1 sont compris entre 3,3 et 6,2 % | | | |
| Les % de masquant de l'ex. 3 sont compris entre 6,5 et 8,2 % | | | |
| Les % de masquant de l'ex. 6 sont compris entre 5,4 et 7,7 % | | | |

On procède alors à un test gustatif de chacun de ces mélanges dans 30 ml d'eau.
Exemple 14 - Contient 500 mg de chlorhydrate de quinine + 23,56 mg du masquant
Exemple 15 - Contient 500 mg de chlorhydrate de quinine + 36,65 mg du masquant
Exemple 16 - Contient 500 mg de chlorhydrate de quinine + 36,48 mg du masquant

Les résultats obtenus sont les suivants :

Exemple 14 : goût sucré et légèrement amer apparaissant en même temps, puis l'amertume diminue et disparaît après 3 minutes 45 secondes, tandis que le goût sucré se prolonge jusqu'à 4 minutes 25 secondes.

Exemple 14 : goût sucré agréable et légèrement amer ; l'amertume diminue et disparaît après 2 minutes 30 secondes, tandis que le goût sucré se prolonge jusqu'à 3 minutes 40 secondes.

Exemple 16 : goût sucré accompagné d'une flaveur de réglisse et de caramel et d'une légère amertume, plus ronde que dans les tests des exemples 13 et 14 ; l'amertume diminue et disparaît après 3 minutes, puis le goût sucré se prolonge pendant 4 minutes 30 secondes

Là aussi, cette comparaison démontre clairement les effets des masquants selon l'invention.

On procède de même avec du dextromethorphane, un antittussif qui a, lui aussi, un goût amer

| | **Exemple 17 %** | **Exemple 18 %** | **Exemple 19 %** | **Exemple 20 %** |
|---|---|---|---|---|
| Dextromethorphane | 94,2 | 95,5 | 96,0 | 91,2 |
| Masquant de l'exemple 2 | 5,8 | | | |
| Masquant de l'exemple 4 | | 4,5 | | |
| Masquant de l'exemple 7 | | | 4,0 | |
| Masquant de l'exemple 8 | | | | 8,8 |
| Les % de masquant de l'ex. 2 sont compris entre 4,2 et 6,5 % | | | | |
| Les % de masquant de l'ex. 4 sont compris entre 3,5 et 5,5 % | | | | |
| Les % de masquant de l'ex. 7 sont compris entre 3,2 et 5,0 % | | | | |
| Les % de masquant de l'ex. 8 sont compris entre 7,0 et 9,5 %. | | | | |

Au goût amer désagréable d'un médicament contenant cet antitussif s'est substitué un goût sucré, dont la note générale est différente selon les exemples.

L'invention a été donnée ici en exemple pour la masquage de goûts amers, qui sont les perceptions les plus désagréables et les plus difficiles à masquer. Elle s'applique a fortiori à d'autres perceptions gustatives ou olfactives, généralement moins agressives, telles que les goûts acides et salés.

## Revendications

1. Masquant en poudre pour goûts pharmaceutiques, comprenant un mélange entre un agent de sapidité et un potentiateur, **caractérisé en ce que** ce mélange se présente sous forme d'un mélange intime dont la répartition agent de sapidité/potentiateur est sensiblement homogène, non statistique, et dont la proportion de l'agent de sapidité par rapport au potentiateur est sensiblement constante et égale d'une particule de poudre à l'autre et **en ce que** la granulométrie de la poudre présente une répartition gaussienne, laquelle est comprise entre 10 et 100 µm.

2. Masquant selon la revendication 1, **caractérisé en ce que** la proportion agent de sapidité/potentiateur(s) est comprise entre 97/3 et 90/10, valeurs exprimées en parties pondérales.

3. Masquant selon la revendication 1, **caractérisé en ce que** l'agent de sapidité comprend un édulcorant.

4. Masquant selon la revendication 1, 2 ou 3, **caractérisé en ce que** l'agent de sapidité est choisi dans le groupe comprenant les saccharinates de sodium ou de calcium, la saccharine, l'aspartyl-phénylalanine, l'acésulfame, les cyclamates et le stévioside, et leurs mélanges, et /ou que le potentiateur est choisi dans le groupe comprenant la thaumatine, la néohespéridine dihydrochalcone (NHDC), la glycyrrhizine, et leurs mélanges.

5. Procédé de préparation du masquant selon l'une des revendications précédentes, **caractérisé en ce qu'**on prépare séparément une solution d"agent de sapidité et une solution de potentiateur, puis qu'on mélange les deux solutions en ajoutant la solution de potentiateur dans la solution d'agent de sapidité, puis qu'on pulvérise le mélange en solution obtenu par atomisation séchage pour obtenir une poudre.

6. Utilisation du masquant selon l'une des revendications 1 à 4 pour masquer les goûts désagréables des produits pharmaceutiques.

7. Médicament comprenant un principe actif et un masquant selon l'une des revendications 1 à 4 et, le cas échéant, un excipient pharmacologiquement acceptable.

8. Médicament pulvérulent, **caractérisé en ce qu'**il se présente sous forme d'un mélange intime dont la répartition agent de sapidité/potentiateur/principe actif est sensiblement homogène, non statistique, et dont les proportions agent de sapidité/potentiateur/principe actif sont sensiblement constantes et égales d'une particule de poudre à l'autre, et **en ce que** la granulométrie de la poudre présente une répartition gaussienne, laquelle est comprise entre 10 et 100 µm.

9. Médicament selon la revendication 8, **caractérisé en ce que** la proportion agent de sapidité/potentiateur(s) est comprise entre 97/3 et 90/10, valeurs exprimées en parties pondérales.

10. Médicament selon la revendication 8, **caractérisé en ce que** l'agent de sapidité comprend un édulcorant.

11. Médicament selon la revendication 8, 9 ou 10, **caractérisé en ce que** l'agent de sapidité est choisi dans le groupe comprenant les saccharinates de sodium ou de calcium, la saccharine, l'aspartyl-phénylalanine, l'acésulfame, les cyclamates et le stévioside, et leurs mélanges, et /ou que le potentiateur est choisi dans le groupe comprenant la thaumatine, la néohespéridine dihydrochalcone (NHDC), la glycyrrhizine, et leurs mélanges.

12. Médicament en granulés, comprimés , comprimés effervescents, cachets, pastilles et gommes à mâcher obtenus à partir du médicament selon l'une des revendications 7 ou 8.

## Claims

1. Powdered masking agent for pharmaceutical tastes, comprising a mixture between a flavour modifier and a potentiator, **characterized in that** this mixture is provided in the form of an intimate mixture in which the flavour modifier/potentiator distribution is essentially homogeneous and non-random, and in which the proportion of the flavour modifier relative to the potentiator is substantially constant and equal from one powder particle to another and **in that** the particle size distribution of the powder exhibits a Gaussian distribution, which is between 10 and 100 µm.

2. Masking agent according to Claim 1, **characterized in that** the flavour modifier/potentiator(s) ratio is between 97/3 and 90/10, values expressed in parts by weight.

3. Masking agent according to Claim 1, **characterized in that** the flavour modifier comprises a sweetener.

4. Masking agent according to Claim 1, 2 or 3, **characterized in that** the flavour modifier is chosen from the group comprising sodium or calcium saccharinates, saccharine, aspartylphenylalanine, acesulfame, cyclamates and stevioside, and mixtures thereof, and/or **in that** the potentiator is chosen from the group comprising thaumatin, neohesperidin dihydrochalcone (NHDC), glycyrrhizin, and mixtures thereof.

5. Process for preparing the masking agent according to one of the preceding claims, **characterized in that** a flavour modifier solution and a potentiator solution are prepared separately and then the two solutions are mixed by adding the potentiator solution to the flavour modifier solution, and then the mixture of solution obtained is spray-dried in order to obtain a powder.

6. Use of the masking agent according to one of Claims 1 to 4, for masking the unpleasant taste of pharmaceutical products.

7. Medicament comprising an active ingredient and a masking agent according to one of Claims 1 to 4 and, where appropriate, a pharmacologically acceptable excipient.

8. Pulverulent medicament, **characterized in that** it is provided in the form of an intimate mixture in which the flavour modifier/potentiator/active ingredient distribution is essentially homogeneous and non-random, and in which the flavour modifier/potentiator/active ingredient ratios are substantially constant and equal from one powder particle to another and **in that** the particle size distribution of the powder exhibits a Gaussian distribution, which is between 10 and 100 µm.

9. Medicament according to Claim 8, **characterized in that** the flavour modifier/potentiator(s) ratio is between 97/3 and 90/10, values expressed in parts by weight.

10. Medicament according to Claim 8, **characterized in that** the flavour modifier comprises a sweetener.

11. Medicament according to Claim 8, 9 or 10, **characterized in that** the flavour modifier is chosen from the group comprising sodium or calcium saccharinates, saccharine, aspartylphenylalanine, acesulfame, cyclamates and stevioside, and mixtures thereof, and/or **in that** the potentiator is chosen from the group comprising thaumatin, neohesperidin dihydrochalcone (NHDC), glycyrrhizin, and mixtures thereof.

12. Medicament in the form of granules, tablets, effervescent tablets, cachets, pastilles and chewing gums obtained from the medicament according to either of Claims 7 and 8.

## Patentansprüche

1. Pulverförmiges Maskierungsmittel für pharmazeutische Geschmäcke, ein Gemisch eines Geschmacksstoffes und eines Verstärkers umfassend und **dadurch gekennzeichnet, dass** sich dieses Gemisch in Gestalt eines innigen Gemischs darbietet, dessen Geschmacksstoff/Verstärker-Verteilung im Wesentlichen homogen und nicht statistisch ist und dessen Anteil von Geschmacksstoff im Verhältnis zum Verstärker im Wesentlichen konstant und von einem Pulverteilchen zum nächsten gleichbleibend ist, und dadurch, dass die Korngrössen des Pulvers eine Gaussschen Verteilung mit Werten zwischen 10 und 100 µm aufweisen.

2. Maskierungsmittel gemäss Anspruch 1, **dadurch gekennzeichnet, dass** das Maskierungsmittel-Verstärker-Verhältnis zwischen 97 : 3 und 90 : 10 liegt, wobei die Werte in Gewichtsteilen ausgedrückt sind.

3. Maskierungsmittel gemäss Anspruch 1, **dadurch gekennzeichnet, dass** das Maskierungsmittel einen Süssstoff umfasst.

4. Maskierungsmittel gemäss Anspruch 1, 2 oder 3, **dadurch gekennzeichnet, dass** der Geschmacksstoff aus der Gruppe ausgewählt ist, die die Saccharinate von Natrium oder Calcium, das Saccharin, das Aspartylphenylalanin, das Acesulfam, die Cyclamate und das Steviosid sowie ihre Mischungen umfasst, und/oder der Verstärker aus der Gruppe ausgewählt ist, die das Thaumatin, das Neohesperidin-Dihydrochalcon (NHDC), das Glycyrrhizin und ihre Mischungen umfasst.

5. Verfahren zur Herstellung des Maskierungsmittels gemäss einem der vorangehenden Ansprüche, **dadurch gekennzeichnet, dass** man eine Lösung von Geschmacksstoff und eine Lösung von Verstärker getrennt herstellt, dann diese zwei Lösungen vermischt, indem man die Verstärkerlösung der Geschmacksstofflösung hinzufügt, dann das erhaltene gelöste Gemisch durch Sprühtrockung pulverisiert, um ein Pulver zu erhalten.

6. Verwendung des Maskierungsmittels gemäss einem der Ansprüche 1 bis 4, um die unangenehmen Geschmäcke von pharmazeutischen Erzeugnissen zu maskieren.

7. Medikament mit einem Wirkstoff und einem Maskierungsmittel gemäss einem der Ansprüche 1 bis 4 sowie gegebenenfalls einem pharmakologisch annehmbaren Bindemittel.

8. Pulverförmiges Medikament, **dadurch gekennzeichnet, dass** es sich in Gestalt eines innigen Gemisches darbietet, dessen Verteilung von Geschmacksstoff, Verstärker und Wirkstoff im Wesentlichen homogen und nicht statistisch ist und dessen Verhältnisse von Geschmacksstoff, Verstärker und Wirkstoff im Wesentlichen konstant und von einem Pulverteilchen zum nächsten gleichbleibend sind, und dadurch, dass die Komgrössen des Pulvers eine Gaussschen Verteilung mit Werten zwischen 10 und 100 µm aufweisen.

9. Medikament gemäss Anspruch 8, **dadurch gekennzeichnet, dass** das Geschmacksstoff-Verstärker-Verhältnis zwischen 97 : 3 und 90 : 10 liegt, wobei die Werte in Gewichtsteilen ausgedrückt sind.

10. Medikament gemäss Anspruch 8, **dadurch gekennzeichnet, dass** der Geschmacksstoff einen Süssstoff umfasst.

11. Medikament gemäss Anspruch 8, 9 oder 10, **dadurch gekennzeichnet, dass** der Geschmacksstoff aus der Gruppe ausgewählt ist, die die Saccharinate von Natrium oder Calcium, das Saccharin, das Aspartylphenylalanin, das Acesulfam, die Cyclamate und das Steviosid sowie ihre Mischungen umfasst, und/oder der Verstärker aus der Gruppe ausgewählt ist, die das Thaumatin, das Neohesperidin-Dihydrochalcon (NHDC), das Glycyrrhizin und ihre Mischungen umfasst.

12. Medikament in der Gestalt von Körnchen, Tabletten, Brausetabletten, Cachets, Pastillen und Kaugummis, die ausgehend von dem Medikament gemäss einem der Ansprüche 7 oder 8 erhalten werden.
